Europäisches Patentamt

European Patent Office

Office européen des brevets

(11) Veröffentlichungsnummer: **0 013 286**
**B1**

(12) **EUROPÄISCHE PATENTSCHRIFT**

(45) Veröffentlichungstag der Patentschrift:
04.11.81

(21) Anmeldenummer: 79102929.1

(22) Anmeldetag: 13.08.79

(51) Int. Cl.³: **B 01 J 23/58**, B 01 J 23/54,
B 01 J 37/02, C 07 C 7/163,
C 07 C 7/167, C 07 C 5/02

(54) Hydrierkatalysator, seine Herstellung und Verwendung.

(30) Priorität: 11.11.78 DE 2849026

(43) Veröffentlichungstag der Anmeldung:
23.07.80 Patentblatt 80/15

(45) Bekanntmachung des Hinweises auf die Patenterteilung:
04.11.81 Patentblatt 81/44

(84) Benannte Vertragsstaaten:
AT BE DE FR GB IT NL SE

(56) Entgegenhaltungen:
GB-A-1 283 737
DE-A-2 025 411

(73) Patentinhaber: BAYER AG, Zentralbereich Patente,
Marken und Lizenzen, D-5090 Leverkusen 1, Bayerwerk
(DE)

(72) Erfinder: Swodenk, Wolfgang, Dr., Auf dem Broich 5,
D-5068 Odenthal (DE)
Erfinder: Lauer, Hubert, Dr., Iltisweg 8, D-4047 Dormagen
(DE)
Erfinder: Birkenstock, Udo, Dr., Schneppersdelle 2,
D-4030 Ratingen 8 (DE)
Erfinder: Schmidt, Herbert, Im Hederichsfeld 52,
D-5090 Leverkusen 3 (DE)

Hydrierkatalysator, seine Herstellung und Verwendung

Die vorliegende Erfindung betrifft einen neuartigen, hochaktiven Edelmetall-Trägerkatalysator sowie seine Herstellung und Verwendung für die Hydrierung von Kohlenwasserstoffen.

Die petrochemischen Grundstoffe Äthylen, Propylen, Butadien, Isopren, Benzol, Toluol und viele andere werden heute weltweit durch thermische oder katalytische Spaltung von Erdgasen oder Erdölfraktionen gewonnen. Die Spaltverfahren erzeugen jedoch neben den gewünschten Kohlenwasserstoffen mehr oder weniger große Mengen an höher ungesättigten Kohlenwasserstoffen, so daß Reinigungsschritte — meist hydrierender Art — allgemein angewendet werden. Diesen hydrierenden Reinigungsverfahren für ungesättigte Kohlenwasserstoffe kommt daher in der gesamten Petrochemie eine große wirtschaftliche Bedeutung zu und die Verfahren werden ausgewählt nach Selektivität, Aktivität, Preis und Lebensdauer der verwendeten Katalysatoren einerseits sowie nach dem Aufwand an Investitionskosten, an Personal und Hilfsenergien für den Betrieb der Verfahren andererseits. Hydrierverfahren für ungesättigte Kohlenwasserstoffe arbeiten in der Gas- oder Flüssigphase, wobei die Gasphase im wesentlichen auf die niedrigsiedenden $C_2$- und $C_3$-Kohlenwasserstoffe begrenzt ist, da bei den höhersiedenden Kohlenwasserstoffen hohe und für die Reaktion ungünstige Temperaturen angewendet werden müssen, um diese Stoffe in der Gasphase zu halten.

Für solche Hydrierung ungesättigter Anteile in Kohlenwasserstoff-Fraktionen werden Nickel-, Kobalt-, Wolfram- oder entsprechende Mischkatalysatoren auf inerten Trägern verwendet. Weiterhin werden wegen ihrer hohen Selektivität und wegen der günstigen Temperatur- und Druckbedingungen, die sie ermöglichen, besonders vorteilhaft Edelmetallkatalysatoren der VIII. Gruppe des Periodensystems angewendet, wie in Chemical Engineering Progress, Vol. 69, No. 5 (1973) S. 65—68, beschrieben. Verwendete Katalysatoren sind beispielsweise gemäß DE-PS 15 18 827 Palladium auf Lithium-Aluminium-Spinell mit inneren Oberflächen bis 100 m²/g, oder auch gemäß US-PS 3 843 137 Palladium auf Aluminiumoxidträgern mit ca. 90 m²/g Oberfläche. In der DT-OS 2 059 978 wird beschrieben, wie man durch Wasserdampf- und Temperatur-Vorbehandlung eines Tonerde-Trägers mit mehr als 120 m²/g Oberfläche und nachfolgende Edelmetallsalzimprägnierung einen Palladium-Katalysator für die Kohlenwasserstoff-Hydrierung erhält. Eine Verbesserung der Eigenschaften von derartigen Edelmetall-Katalysatoren kann auch durch den Zusatz von Promotoren erzielt werden. So werden gemäß DE-OS 2 546 513 seltene Erden einem Katalysator zugemischt, in dem als aktives Material Palladium und als Träger Tonerde/SiO₂ mit einer inneren Oberfläche von mehr als 200 m²/g dienen. In der DE-OS 3 947 510 wird ein Verfahren beschrieben, demzufolge ein Diolefin zum Olefin an einem Palladium-Träger-Katalysator hydriert wird unter Zusatz einer wäßrigen Zinksalzlösung zu dem zu hydrierenden Diolefin.

Es wurde nun ein ein Edelmetall der VIII. Gruppe des Periodensystems auf Aluminiumoxid als Träger enthaltender hochaktiver Hydrierkatalysator gefunden, der dadurch erhältlich ist, daß man ein Aluminiumoxid-Trägermaterial mit einer inneren Oberfläche (BET) von kleiner als 80 m²/g entsprechend seiner Saugfähigkeit bis zur Sättigung mit einer eine anorganische Base enthaltenden wäßrigen Lösung tränkt, im Warmluftstrom auf einen Restfeuchtegehalt von weniger als 2 Gew.-% trocknet, in an sich bekannter Weise Edelmetallsalzlösung auftränkt, wobei für die Tränkung 0,1—50 Grammäquivalent anorganische Base pro Grammäquivalent Edelmetall eingesetzt werden, und daß man den so erhaltenen Katalysator gegebenenfalls reduziert, mit Wassr wäscht und trocknet.

Als Träger zur Herstellung der erfindungsgemäßen Katalysatoren wird ein Aluminiumoxid mit einer BET-Oberfläche von kleiner als 80 m²/g, bevorzugt kleiner als 20 m²/g, ganz besonders bevorzugt kleiner als 10 m²/g, verwendet. Derartige Trägermaterialien weisen im allgemeinen eine Saugfähigkeit von 20—60 ml Wasser pro 100 g Träger auf. Sie liegen meist in Form von Kugeln, Extrudaten oder Tabletten mit einem Durchmesser von 2—8 mm vor.

Als anorganische Basen kommen im allgemeinen Natrium-, Kalium- und Lithiumhydroxid sowie die entsprechenden Carbonate und Bicarbonate in Betracht. Zur Vorbehandlung des Aluminiumoxid-Trägers mit anorganischer wäßriger Baselösung wird zweckmäßigerweise zunächst seine Saugfähigkeit für Wasser bestimmt und die Tränkung dann mit der der maximalen Saugfähigkeit entsprechenden Menge an Baselösung vorgenommen, so daß gerade die Sättigung erreicht wird. Diese Methode hat den Vorteil, daß die aufzutragende Menge genau bestimmt werden kann. Die Konzentration der Baselösung richtet sich zweckmäßigerweise nach der aufzutragenden Menge an anorganischer Base und dieses wiederum wird aus der aufzubringenden Edelmetallmenge bestimmt, wobei pro Grammäquivalent Edelmetall 0,1—50, vorzugsweise 0,1—20 Grammäquivalent Base eingesetzt werden. Ganz bevorzugt wählt man das Verhältnis 0,1—5 Grammäquivalent Base pro Grammäquivalent Edelmetall.

Die Trocknung des Trägers im Warmluftstrom geschieht nach üblichen Methoden in fest angeordneten Betten, durch die der Warmluftstrom geführt wird. Die Trocknung auf weniger als 2 Gew.-% Restfeuchtigkeit erfolgt bis zu Gewichtskonstanz und ist ein wesentlicher Schritt zur Erzielung des erfindungsgemäßen Hydrierkatalysators. Der Warmluftstrom besitzt zweckmäßigerweise eine Temperatur von 80—150° C. Es werden Gehalte an Restfeuchtigkeit von kleiner als 2 Gew.-%, bevorzugt von kleiner als 1 Gew.-%, erhalten. Diese absoluten Restfeuchtigkeitsgehalte entsprechen Werten von kleiner als 10%, bevorzugt kleiner als 4%, besonders bevorzugt kleiner als 1% der Saugfähigkeit des Trägers.

Als Edelmetalle kommen für die erfindungsgemäßen Katalysatoren die entsprechenden Elemente der VIII. Gruppe des Periodensystems in Betracht, z. B. Palladium, Platin, Rhodium, Iridium. Bevorzugt werden Palladium und Platin als katalytisch wirksamer Anteil verwendet. Ganz besonders geeignet ist Palladium. Das Edelmetall wird als Lösung eines handelsüblichen Salzes auf dem vorgehandelten Träger aufgebracht.

Für die Auftragung des Edelmetallsalzes wird vorteilhafterweise die bereits beschriebene Methode verwendet, wonach die das Edelmetall in Form eines handelsüblichen Salzes enthaltende Flüssigkeitsmenge so bemessen wird, daß sie der Saugfähigkeit des basebehandelten Trägers bis zu einer Sättigung entspricht. Andere Auftragsmethoden wie Spritzüberziehen oder Überschichten mit Edelmetallsalzlösung sind ebenfalls möglich. Die Konzentration der Metallsalzlösung wird so eingestellt, daß der fertige Katalysator einen Gehalt von bis zu 10 g, bevorzugt bis zu 5 g Edelmetall pro 1 Liter Träger enthält.

Als Edelmetall wird bevorzugt Palladium, im allgemeinen in der Salzform $Na_2[PdCl_4]$, oder auch als anderes Salz, verwendet. Im allgemeinen ist nach einer Wartezeit von ca. 30 Minuten die Auftränkung des Edelmetalls erfolgt, und der Katalysator kann, je nach der beabsichtigten Verwendungsform, dierekt eingesetzt oder nach bekannten Methoden mit Hydrazin, Formaldehyd oder Wasserstoff reduziert, mit Wasser gewaschen und getrocknet werden.

Die erfindungsgemäßen Katalysatoren sind für die Hydrierung von ungesättigten Anteilen in Kohlenwasserstoffgemischen aller Art geeignet. Die Erfindung betrifft infolgedessen auch die Verwendung der neuen Katalysatoren für die Hydrierung von 3 bis 10 Kohlenstoffatome enthaltenden ungesättigten Kohlenwasserstoffen. Bevorzugt gelangen sie zur Hydrierung von Acetylenen, Diolefinen und Olefinen in Kohlenwasserstoffen vom $C_3$- bis $C_{10}$-Siedebereich oder in Kohlenwasserstoffgemischen mit 3 bis 10 Kohlenstoffatomen zur Anwendung. Sie sind für Hydrierungen in der Flüssig- oder Rieselphase geeignet. Durch den niedrigen Edelmetallgehalt sowie durch die einfache Art der Herstellung, die weder eine aufwendige Trägerbehandlung noch eine Dotierung teurer oder schwer aufzutragender Promotoren erforderlich macht, werden besonders preisgünstige Katalysatoren zur Verfügung gestellt. Aktivität und Selektivität der erfindungsgemäßen Katalysatoren sind dabei ungewöhnlich hoch, so daß hohe Produktdurchsätze bei kleinen Katalysator- und Reaktorvolumina erzielt werden. Die Aktivität der erfindungsgemäß hergestellten Katalysatoren ist im technischen Betrieb über mehrere tausend Betriebsstunden nahezu konstant. Falls erforderlich kann nach extrem langer Betriebszeit die Aktivität durch einfaches Erhitzen in an sich bekannter Weise in Luft auf 500° C wiederhergestellt werden, so daß der Katalysator erneut verwendbar ist. Hervorzuheben ist die hohe Flexibilität der erfindungsgemäßen Katalysatoren im Einsatz: Nicht nur können ungesättigte Kohlenwasserstoffe mit 3—10 Kohlenstoffatomen hydriert werden, es können auch acetylenische Verunreinigungen im angegebenen $C_3$- bis $C_{10}$-Siedebereich ohne Veränderung der diolefinischen und olefinischen Kohlenwasserstoffen selektiv hydriert werden, beispielsweise Methylacetylen und Propadien zu Propylen. Man kann weiterhin auch acetylenische und diolefinische Kohlenwasserstoffe selektiv hydrieren, ohne daß es zur Hydrierung der olefinischen Anteile kommt. Zum Beispiel können Vinylacetylen, Äthylacetylen und Butadien-1,3 als ungesättigte Bestandteile beispielsweise einer $C_4$-Fraktion aus der thermischen Spaltung von Naphtha- oder Leichtbenzin so hydriert werden, daß Vinylacetylen und Äthylacetylen quantitativ in n- und i-Butan umgewandelt werden und das Butadien-1,3 praktisch vollständig selektiv in n- und i-Buten überführt wird. Es können im Bedarfsfall auch alle acetylenischen, diolefinischen und olefinischen Komponenten zu den entsprechenden Paraffinen hydriert werden.

Ein weiterer nicht zu unterschätzender Vorteil des erfindungsgemäßen Katalysators ist seine sehr geringe Bildung an höher siedenden Nebenprodukten, die bei ähnlichen Hydrierverfahren dem Fachmann unter der Bezeichnung »Green Oil« bekannt sind und dort wegen ihrer hohen Bildung oft eine sogenannte Öl-Wäsche des hydrierten Produktes erforderlich machen. Die Green-Oil-Bildung verringert somit nicht nur die Ausbeute der gewünschten Produkte, sondern macht das Verfahren auch aufwendig und teuer.

Die folgenden Beispiele sollen den erfindungsgemäßen Katalysator und seine Verwendung näher erläutern.

### Beispiel 1

#### a) Katalysatorherstellung

Ein Liter eines Aluminiumoxidträgers, bestehend aus 3 mm Strangpreßlingen, mit einer BET-Oberfläche von 4 m²/g, einer Saugfähigkeit von 28 ml $H_2O$ pro 100 g Träger und einem Schüttgewicht von 1161 g/l wurde mit 325 ml einer wäßrigen NaOH-Lösung imprägniert, die 1,2 g, entsprechend 0,03 Grammäquivalent NaOH enthielt. Die Lösung wurde innerhalb von 2 Minuten völlig vom Träger angesaugt. Der feuchte Träger wurde in ein senkrechtes Glasrohr von ca. 2 l Inhalt geschüttet und dann mit warmer Luft von 105 bis 120°C in einer Menge von 20 m³/h von unten nach oben behandelt. Es wurde bis zur Gewichtskonstanz des Trägers getrocknet, was nach 30 Minuten der Fall war. Der Restfeuchtegehalt des getrockneten Trägers lag nach Abkühlen auf Raumtemperatur bei 0,11 Gew.-%, entsprechend ca. 0,39% der Saugfähigkeit.

Der vorbehandelte, trockene Träger wurde seiner Saugfähigkeit entsprechend mit einer wäßrigen Natriumtetrachloropalladat-Lösung, 2 g Pd entsprechend 0,037 Grammäquivalent enthaltend, getränkt und 15 Minuten stehengelassen. Zur Reduktion des Palladiums wurde der Träger in einem Becherglas mit 400 ml einer 10%igen wäßrigen Hydrazinhydrat-Lösung überschichtet. Dann wurde der Katalysator in einem fließenden Strom von destilliertem Wasser gewaschen, bis keine Natrium- und Chloridionen und kein Hydrazin im Waschwasser mehr nachweisbar waren, was nach 10 Stunden der Fall war. Die anschließende Trocknung geschah im Warmluftstrom wie vorher bei der Trocknung des Trägers beschrieben. Der so hergestellte Katalysator enthielt 2 g Pd pro Liter Träger (~0,172 Gew.-%).

### b) Anwendung

Der Katalysator wurde zur selektiven Hydrierung acetylenischer Verunreinigungen in einem Propylenstrom der in Tabelle I, Spalte 1 angegebenen Zusammensetzung, wie er aus thermischen Spaltanlagen von Naphtha oder Leichtbenzin erhältlich ist, eingesetzt.

Dazu wurden 600 ml des nach der obigen Vorschrift hergestellten Katalysators in ein senkrecht angeordnetes Stahlrohr von 180 cm Länge und 2,5 cm innerem Durchmesser eingefüllt. Das Rohr besaß einen Kühlmantel, der mit Kühlwasser von 20°C beschickt wurde. Nach unten hatte das Rohr freien Ablauf in ein Abscheidegefäß. Am oberen Ende des Rohres waren zwei Anschlußstutzen angebracht zur Dosierung des Propylenstromes und des Hydrierwasserstoffs. Über einen Anschlußstutzen wurde 12 kg des flüssigen Propylenstromes pro Stunde kontinuierlich eindosiert, über das zweite Anschlußstück wurde Wasserstoff aus einer Stahlflasche zugegeben und ein solcher Druck im Reaktionsrohr und Abscheidegefäß aufrechterhalten, daß das hydrierte Produkt frei von acetylenischen Verbindungen erhalten wurden. Das hydrierte Produkt wurde im unteren Abscheidegefäß aufgefangen und kontinuierlich über ein Entspannungsventil daraus entfernt, so daß immer ein konstanter Flüssigkeitsstand darin gehalten wurde. In regelmäßigen Abständen wurden Proben entnommen, die gaschromatographisch auf ihre Zusammensetzung untersucht wurden. Nach 1000 Betriebsstunden betrug der zur völligen Entfernung der acetylenischen Verunreinigungen erforderliche Reaktordruck, der über die zugeführte Wasserstoffmenge eingestellt wurde, 12 bar absolut.

Die Zusammensetzung des hydrierten Produktes nach 1000 Betriebsstunden ist in Tabelle I, Spalte 2, aufgeführt. Es ist ersichtlich, daß die acetylenischen Verunreinigungen Methylacetylen und Propadien mit hoher Selektivität zu Propylen hydriert werden, und daß nur 0,15 Gew.-% Dimere gebildet werden.

Tabelle I

| | Komponenten in Gew.-% | | |
| | 1 | 2 | 3 |
| --- | --- | --- | --- |
| Propan | 3,3 | 4,1 | 4,3 |
| Propylen | 93,5 | 95,75 | 95,21 |
| Methylacetylen | 1,8 | 0,003 | 0,003 |
| Propadien | 1,4 | 0,003 | 0,003 |
| Dimere | — | 0,15 | 0,45 |
| Polymere | — | — | 0,04 |

### Beispiel 2 (zum Vergleich)

#### a) Katalysatorherstellung

Es wurde ein Aluminiumoxidträger mit einer BET Oberfläche von 350 m²/g, einem Schüttgewicht von 827 g/l und einer Saugfähigkeit von 44,2 ml H₂O/100 g Träger verwendet. Daraus wurde ein Katalysator genau nach der Vorschrift von Beispiel 1 hergestellt. Entsprechend der geänderten Saugfähigkeit wurde gegenüber Beispiel 1 ein Liter des o. a. Trägers mit 365 ml einer wäßrigen NaOH-Lösung, die 1,2 g entsprechend 0,03 Grammäquivalent NaOH enthielt, imprägniert. Der so imprägnierte und gemäß Beispiel 1 getrocknete Träger wurde seiner Saugfähigkeit entsprechend mit einer wäßrigen Natriumtetrachloropalladat-Lösung 2 g entsprechend 0,037 g Äquivalent Pd enthaltend, getränkt und gemäß Beispiel 1 weiterverarbeitet. Der fertige Träger enthielt, wie in Beispiel 1 beschrieben, 2 g Pd pro Liter Träger (~0,24 Gew.-%).

#### b) Anwendung

Der Katalysator wurde in derselben Apparatur und mit demselben Propylenstrom wie in Beispiel 1 zur Hydrierung acetylenischer Verunreinigungen eingesetzt. Hier betrug nach 1000 Betriebsstunden der für die völlige Hydrierung erforderliche Druck 13 bar. Die Zusammensetzung des Hydrierproduktes nach dieser Betriebszeit ist in Tabelle I, Spalte 3, aufgeführt. Es ist ersichtlich, daß der Katalysator ähnlich aktiv und selektiv wie der im Beispiel 1 beschriebene ist. Die Dimerenbildung ist jedoch mit 0,45 Gew.-% dreimal so hoch wie im Beispiel 1 und es werden zusätzlich Polymere in einer Menge von 0,04 Gew.-% gebildet.

### Beispiel 3

Der Katalysator von Beispiel 1 wurde in derselben Apparatur wie in Beispiel 1 für die Hydrierung einer Butadien-reichen $C_4$-Fraktion, wie sie bei der thermischen Spaltung von Naphtha oder Leichtbenzin anfällt, eingesetzt. Die Zusammensetzung dieser Fraktion ist in Tabelle II, Spalte 1, aufgeführt. Aufgabe der Hydrierung war, was Butadien dieses Stromes selektiv zu n-Buten zu hydrieren. Dazu wurden in die in Beispiel 1 beschriebenen Hydrierapparatur 4 kg/h der flüssigen $C_4$-Fraktion mit der in Tabelle II, Spalte 1, angegebenen Zusammensetzung eindosiert. Aus dem Abscheidegefäß wurden 8 kg/h des hydrierten Produktes zur Verdünnung des Einsatzproduktes als Rückführstrom zusammen mit der $C_4$-Fraktion auf den Kopf des Reaktionsrohres gegeben. Der Druck des Reaktionssystems wurde über die Wasserstoffzudosierung wieder so hoch eingestellt, daß eine völlige Butadienentfernung erzielt wurde. Nach 1000 Betriebsstunden betrug der dazu erforderliche Druck 15 bar absolut. Die dabei erzielte Zusammensetzung des hydrierten Produktes ist aus Tabelle II, Spalte 2, ersichtlich. Das Butadien wurde mit hoher Selektivität zu Buten hydriert. Die Dimerenbildung lag bei nur 0,03 Gew.-%.

Tabelle II

|  | Komponenten in Gew.-% | | |
|---|---|---|---|
|  | 1 | 2 | 3 |
| n + i-Butan | 5,4 | 6,1 | 10,5 |
| n + i-Buten | 49,24 | 93,77 | 89,37 |
| Butadien-1,3 | 44,3 | 0,1 | 0,1 |
| Vinylacetylen | 0,87 | — | — |
| Äthylacetylen | 0,19 | — | — |
| Dimere | — | 0,03 | 0,03 |

### Beispiel 4 (zum Vergleich)

#### a) Katalysatorherstellung

Unter Verwendung des Aluminiumoxidträgers aus Beispiel 1 mit 4 m²/g BET Oberfläche wurde ein Katalysator ohne Basevorbehandlung des Trägers wie folgt hergestellt:
1 l des Trägers wurde mit 325 ml einer wäßrigen Natriumtetrachloropalladat-Lösung, 6 g Pd enthaltend, imprägniert. Nach 15 Minuten Wartezeit wurde der Katalysator wie in Beispiel 1 beschrieben mit einer wäßrigen Hydrazinlösung reduziert, mit Wasser gewaschen und getrocknet. Der so hergestellte Katalysator enthielt 6 g Palladium/l Träger ( ~ 0,51 Gew.-%).

#### b) Anwendung

Der Katalysator wurde, genau wie in Beispiel 3 beschrieben, für die Hydrierung der dort angegebenen $C_4$-Fraktionen eingesetzt.
Nach 1000 Betriebsstunden mußte der Druck von 22 bar absolut zur Erzielung der Butadienentfernung eingestellt werden, wobei ein hydriertes Produkt der Zusammensetzung gemäß Tabelle II, Spalte 3, erhalten wurde.
Es ist ersichtlich, daß dieser nach dem Stand der Technik ohne Basevorbehandlung des Trägers hergestellte Katalysator, der wesentlich mehr Palladium als in Beispiel 3 bzw. Beispiel 1 beschriebene Katalysator enthält, spürbar inaktiver und unselektiver arbeitet, was insbesondere daraus entnommen werden kann, daß der erforderliche Hydrierdruck wesentlich höher und die Überhydrierung des Butadiens zum Butan wesentlich stärker war als in Beispiel 3.

### Beispiel 5

#### a) Katalysatorherstellung

Ein Liter eines Aluminiumoxidträgers, bestehend aus Kugeln mit einem Durchmesser von 2,4—4,0 mm, einer BET-Oberfläche von 74 m²/g, einer Saugfähigkeit von 54 ml $H_2O$ pro 100 g Träger und einem Schüttgewicht von 717 g/l, wurde mit 387 ml einer wäßrigen NaOH-Lösung imprägniert, die 6,0 g, entsprechend 0,15 Grammäquivalent NaOH enthielt. Die Lösung wurde innerhalb von 2 Minuten völlig vom Träger aufgesaugt. Der feuchte Träger wurde in ein senkrechtes Glasrohr von ca. 2 l Inhalt geschüttet und dann mit warmer Luft von 105—120° C in einer Menge von 20 m³/h von unten nach oben behandelt. Es wurde bis zur Gewichtskonstanz des Trägers getrocknet, was nach 30 Minuten der Fall war. Der Restfeuchtegehalt des getrockneten Trägers lag nach Abkühlen auf Raumtemperatur bei 0,25 Gew.-%, entsprechend ca. 0,47% der Saugfähigkeit.
Der vorbehandelte, trockene Träger wurde seiner Saugfähigkeit entsprechend mit einer wäßrigen Natriumtetrachloropalladat-Lösung, 5 g Pd entsprechend 0,094 Grammäquivalent enthaltend, getränkt und 15 Minuten stehengelassen. Dann wurde der Katalysator in einem fließenden Strom von destilliertem Wasser gewaschen bis keine Natrium- und Chloridionen im Waschwasser nachweisbar waren, was nach 12 Stunden der Fall war.
Die anschließende Trocknung geschah im Warmluftstrom, wie vorher bei der Trocknung des Trägers beschrieben. Der so hergestellte Katalysator enthielt, als Metall berechnet, 5 g Pd pro Liter Träger ( ~ 0,691 Gew.-%).

b) Anwendung

Der Katalysator wurde in derselben Apparatur wie in Beipsiel 1 für die Hydrierung von Pyrolysebenzin, wie es bei der thermischen Spaltung von Naphtha oder Leichtbenzin anfällt, eingesetzt. Die Zusammensetzung dieses Produktes ist in Tabelle III, Spalte i, aufgeführt. Aufgabe dieser Hydrierung war es, den Dien-Gehalt des Pyrolysebenzins soweit zu erniedrigen, daß ein alterungsstabiles und für die Verwendung als Vergaserkraftstoff spezifikationsgerechtes Hydrierprodukt erhalten wurde. Dazu wurde in die in Beispiel 1 beschriebene Hydrierapparatur 3 kg/h des Pyrolysebenzins mit der in Tabelle III, Spalte 1, angegebenen Zusammensetzung eindosiert. Der Druck des Reaktionssystems wurde über die Wasserstoffzudosierung auf 28 bar eingestellt. Der Kühlmantel des Reaktorrohres wurde mit Kühlwasser von 55°C beschickt. Das Hydrierprodukt wurde aus dem unteren Abscheidegefäß kontinuierlich entnommen.

Die Zusammensetzung des Hydrierproduktes nach 1000 Betriebsstunden ist in Tabelle III, Spalte 2, aufgeführt. Es ist ersichtlich, daß ein praktisch dien-freies und alterungsstabiles Produkt erzielt wurde, und daß keine Neubildung von Existent gum stattfand.

Tabelle III

|  | 1 | 2 |
|---|---|---|
| Bromzahl, g Br/100 g | 68 | 22 |
| Dien-Gehalt, Gew.-% | 17 | 0,5 |
| Existent gum, mg/100 ml | 2 | 2 |
| Potential gum, mg/100 ml | 6000 | 3 |
| Induktionszeit, min. | <60 | >360 |

**Patentansprüche**

1. Hydrierkatalysator, enthaltend ein Edelmetall der VIII. Gruppe des Periodensystems auf Aluminiumoxid als Träger, dadurch erhältlich, daß man ein Aluminiumoxid-Trägermaterial mit einer inneren Oberfläche (BET) von kleiner als 80 $m^2/g$ entsprechend seiner Saugfähigkeit bis zur Sättigung mit einer eine anorganische Base enthaltenden wäßrigen Lösung tränkt, im Warmluftstrom auf einen Restfeuchtegehalt von weniger als 2 Gew.-% trocknet, in an sich bekannter Weise Edelmetallsalzlösung aufttränkt, wobei für die Tränkung 0,1—50 Grammäquibalent anorganische Base pro Grammäquivalent Edelmetall eingesetzt werden, und daß man den so erhaltenen Katalysator gegebenenfalls reduziert, mit Wasser wäscht und trocknet.

2. Verfahren zur Herstellung eines ein Edelmetall der VIII. Gruppe des Periodensystems enthaltenden Katalysators auf Aluminiumoxid als Träger, dadurch gekennzeichnet, daß man ein Aluminiumoxid-Trägermaterial mit einer inneren Oberfläche (BET) von kleiner als 80 $m^2/g$, entsprechend seiner Saugfähigkeit bis zur Sättigung mit einer eine anorganische Base enthaltenden wäßrigen Lösung tränkt, im Warmluftstrom auf einen Restfeuchtegehalt von weniger als 2 Gew.-% trocknet, in an sich bekannter Weise Edelmetallsalzlösung aufttränkt, wobei für die Tränkung 0,1—50 Grammäquivalent anorganische Base pro Grammäquivalent Edelmetall eingesetzt werden und daß man den so erhaltenen Katalysator gegebenenfalls reduziert, mit Wasser wäscht und trocknet.

3. Verwendung des Katalysators gemäß Anspruch 1 für die Hydrierung von 3 bis 10 Kohlenstoffatome enthaltenden ungesättigten Kohlenwasserstoffen.

4. Verwendung des Katalysators gemäß Anspruch 3 für die Selektivhydrierung von Acetylenen, Olefinen und Diolefinen, die in Kohlenwasserstoffen des $C_3—C_{10}$-Siedebereichs oder in Kohlenwasserstoffgemischen mit 3 bis 10 Kohlenstoffatomen enthalten sind.

**Claims**

1. Hydrogenation catalyst containing a noble metal of group VIII of the periodic table on aluminium oxide as the support, which is obtainable by impregnating an aluminium oxide support material with an internal surface area (BET) of less than 80 $m^2/g$, in accordance with its absorbency, to saturation with an aqueous solution containing an inorganic base, drying the material in a steam of warm air to a residual moisture content of less than 2% by weight and impregnating it in a manner which is in itself known with a noble metal salt solution, 0,1—50 gram equivalents of inorganic base being employed per gram equivalent of noble metal for impregnation, and subjecting the catalyst thus obtained to optional reduction, washing with water and drying.

2. Process for the preparation of a catalyst containing a noble metal of group VIII of the periodic table on aluminium oxide as the support, characterised in that an aluminium oxide support material with an internal surface area (BET) of less than 80 $m^2/g$ is impregnated, in accordance with its absorbency, to saturation with an aqueous solution containing an inorganic base, dried in a stream of warm air to a residual moisture content of less than 2% by weight and impregnated in a manner which is in itself known with a noble metal solution, 0,1—50 gram equivalents of inorganic base being emploed per gram equivalent of noble metal for impregnation, and in that the catalyst thus obtained is optionally reduced, washed with water and dried.

3. Use of the catalyst according to Claim 1 for the hydrogenation of unsaturated hydrocarbons containing 3 to 10 carbon atoms.

4. Use of the catalyst according to Claim 1 for the selective hydrogenation of acetylenes, olefines and diolefines which are contained in hydrocarbons of the $C_3$—$C_{10}$-boiling range or in mixtures of hydrocarbons with 3 to 10 carbon atoms.

**Revendications**

1. Catalyseur d'hydrogénation contenant un métal noble du groupe VIII de la Classification Périodique sur un support d'alumine et qu'on obtient en imprégnant la matière de support d'alumine, à une surface spécifique interne (BET) inférieure à 80 m²/g, jusqu'à saturation correspondant à sa capacité d'absorption, par une solution aqueuse contenant une base minérale, en séchant dans un courant d'air jusqu'à une teneur en humidité résiduelle inférieure à 2% en poids, en imprégnant ensuite de manière connue en soi par une solution de sel de métal noble, l'imprégnation étant réalisée à l'aide de 0,1 à 50 équivalents-grammes de base minérale par équivalentgramme de métal noble, et en réduisant le cas échéant le catalyseur obtenu, en le lavant à l'eau et en le séchant.

2. Procédé de préparation d'un catalyseur contenant un métal noble du groupe VIII de la Classification Périodique sur l'alumine servant de support, caractérisé en ce que l'on imprègne une matière de support d'alumine à une surface spécifique interne (BET) inférieure à 80 m²/g, jusqu'à saturation correspondent à sa capacité d'absorption, par une solution aqueuse contenant une base minérale, on sèche dans un courant d'air chaud jusqu'à une teneur en humidité résiduelle inférieure à 2% en poids, on imprègne ensuite de manière connue en soi par une solution de sel de métal noble, l'imprégnation étant réalisée par 0,1 à 50 équivalents-grammes de base minérale par équivalentgramme de métal noble, et le cas échéant on réduit le catalyseur obtenu, on le lave à l'eau et on le cèche.

3. Utilisation du catalyseur selon la revendication 1 pour l'hydrogénation des hydrocarbures insaturés contenant 3 à 10 atomes de carbone.

4. Utilisation du catalyseur selon la revendication 3 pour l'hydrogénation sélective des hydrocarbures acétyléniques, des oléfines et des dioléfines contenus dans les hydrocarbures bouillant dans l'intervalle de $C_3$ à $C_{10}$ ou dans les mélanges d'hydrocarbures contenant de 3 à 10 atomes de carbone.